(19) 

**Europäisches Patentamt**
**European Patent Office**
**Office européen des brevets**

(11) **EP 4 759 375 A1**

(12) **EUROPEAN PATENT APPLICATION**

(43) Date of publication:
**17.06.2026 Bulletin 2026/25**

(21) Application number: **25218227.4**

(22) Date of filing: **25.11.2025**

(51) International Patent Classification (IPC):
***A61Q 5/12*** *(2006.01)*     ***A61K 8/92*** *(2006.01)*
***A61K 8/9789*** *(2017.01)*     ***A61K 8/9794*** *(2017.01)*

(52) Cooperative Patent Classification (CPC):
**A61K 8/922; A61K 8/9789; A61K 8/9794;**
**A61Q 5/12**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **12.12.2024 EP 24219430**

(71) Applicant: **Wella Germany GmbH**
**64295 Darmstadt (DE)**

(72) Inventor: **Akhila, Nair**
**64295 Darmstadt (DE)**

(74) Representative: **Ziebig Hengelhaupt Intellectual**
**Property Attorneys**
**Patentanwaltskanzlei PartGmbB**
**Leipziger Straße 49**
**10117 Berlin (DE)**

(54) **BOTANICAL MIXTURE COMPRISING 9 BOTANICAL COMPONENTS**

(57)     The presently claimed invention relates to the use of a botanical composition (BM) comprising the following 9 botanical components bamboo extract, cactus extract, jojoba oil, safflower oil, olive oil, babassu oil, sesame seed oil, rice bran oil, and evening primrose oil. The presently claimed invention, moreover, is drawn to a hair cosmetic product comprising the botanical composition (BM), a method for treating human hair with the botanical composition (BM) or the hair cosmetic product comprising the botanical composition (BM), and the use of the botanical composition, or the hair cosmetic product for treating human hair, especially for nourishing and or strengthening human hair, or reducing the combing force of human hair.

EP 4 759 375 A1

## Description

### Field of the invention

The presently claimed invention relates to the use of a botanical composition (BM) comprising the following 9 botanical components bamboo extract, cactus extract, jojoba oil, safflower oil, olive oil, babassu oil, sesame seed oil, rice bran oil, and evening primrose oil. The presently claimed invention, moreover, is drawn to a hair cosmetic product comprising the botanical composition (BM), a method for treating human hair with the botanical composition (BM) or the hair cosmetic product comprising the botanical composition (BM), and the use of the botanical composition, or the hair cosmetic product for treating human hair, especially for nourishing and or strengthening human hair, or reducing the combing force of human hair.

### Background of the invention

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with the combing and result in a condition commonly referred to as "fly-away hair" or contribute to an undesirable phenomenon of "split ends".

Further, chemical treatments, such as perming, bleaching, or coloring hair, can also damage hair and leave it dry, rough, lusterless, and damaged. The hair, moreover, can be damaged by ultraviolet radiation or heat.

A variety of approaches have been developed to condition the hair. A common method of providing conditioning benefits to the hair is through the use of conditioning agents such as cationic surfactants and high melting point fatty compounds, silicone compounds, and mixtures thereof.

Especially silicone compounds have been excessively used over the past decades to provide conditioners with enhanced benefits such as hair shine, softness, dry hair smoothness, hair strand alignment (e.g. minimize frizziness), and ease of combing.

However, consumers are becoming more aware of the safety of the ingredients used in their cosmetic products, in particular, the use of synthetic compounds whose toxicology and environmental impact is questionable.

Accordingly, there is a need for keratin fiber treatments which are free of components whose toxicology and environmental impact is questionable.

### Summary of the invention

It is an object of the presently claimed invention to provide a composition that is capable to condition human hair and impart UV and thermal protection to the hair. The composition should preferably exclude synthetic components with uncertain toxicology or potentially harmful environmental impacts.

The object has been solved by a botanical mixture comprising 9 botanical components.

Accordingly, in one aspect, the presently claimed invention is directed to a botanical mixture (BM) comprising the following botanical components

    b1) bamboo extract,
    b2) cactus extract,
    b3) jojoba oil,
    b4) safflower oil,
    b5) olive oil,
    b6) babassu oil,
    b7) sesame seed oil,
    b8) rice bran oil, and
    b9) evening primrose oil.

Surprisingly, it was found that the botanical mixture (BM) is capable of conditioning human hair. Moreover, it was surprisingly found that the botanical mixture (BM) provides one, two or more, or all of the following advantages which are, heat protection up to 230°C, protection against ultraviolet radiation, protection from hair breakage, protection from hair split ends, protection from pollution buildup on the hair, protection from hair frizz. The botanical mixture (BM), moreover, is capable to nourish and moisturize human hair, and to act as moisturize repelling shield. Moreover, the botanical mixture (BM) can reduce the combing force of human hair.

Moreover, it was surprisingly found that the botanical mixture (BM) can be used in a hair cosmetic product like shampoos, conditioners, serums or hair masks and the like to achieve one, two or more, or all of the aforementioned advantages.

The present invention is effective for providing conditioning benefits to hair that is damaged by natural, environmental factors such as shampooing, as well as chemical hair treatments such as bleaching, coloring, or perming.

**Detailed description of the invention**

Botanical mixture (BM)

The botanical mixture (BM) comprises the following 9 botanical components

> b1) bamboo extract,
> b2) cactus extract,
> b3) jojoba oil,
> b4) safflower oil,
> b5) olive oil,
> b6) babassu oil,
> b7) sesame seed oil,
> b8) rice bran oil, and
> b9) evening primrose oil.

In one embodiment the botanical mixture (BM) consists of the 9 botanical components b1), b2), b3), b4), b5), b6), b7), b8), and b9).

The 9 botanical components b1), b2), b3), b4), b5), b6), b7), b8), and b9) are described in more detail hereinafter.

In a preferred embodiment, the botanical mixture (BM) offers versatile benefits tailored to different hair types, making it suitable for a wide range of users. For dry and damaged hair, the oils and extracts provide deep hydration, repair split ends, and restore lost elasticity, leaving hair softer and more manageable. Oily hair benefits from lightweight, non-greasy hydration and scalp-balancing properties that help regulate sebum production without clogging pores. For curly or textured hair, the formulation enhances moisture retention, reduces frizz, and defines curls, providing smoothness and control. Fine hair experiences volumizing effects as the lightweight nature of the ingredients avoids weighing down strands while still providing necessary nourishment. Additionally, for chemically treated or color-treated hair, the antioxidants and reparative compounds protect against further damage, prevent color fading, and reinforce hair strength. This multi-functional approach ensures that the mixture caters to diverse hair needs, enhancing overall health and appearance.

*b1) bamboo extract:*

Bamboo extract (component b1)) is an extract derived from any part of a bamboo plant in the Bambusa genus. Preferably, component b1) is extracted from the leaves, stems, and/or shoots of a Bambusa species, such as preferably Bambusa vulgaris, ensuring a rich concentration of bioactive compounds. Other suitable species of Bambusa in addition to Bambusa vulgaris are Bambusa oldhamii, Bambusa balcooa, Bambusa bambos, Bambusa tulda, Bambusa textilis, Bambusa multiplex, Bambusa arundinacea, Bambusa nutans, Bambusa blumeana, Bambusa tuldoides, Bambusa pervariabilis, Bambusa emeiensis, Bambusa lako, Bambusa dissimulator, Bambusa ventricosa and Bambusa beecheyana.

Examples for suitable extracting agents are water, alcohols and water-alcohol mixtures. Preferred alcohols are $C_{1-6}$-alcohols or mixtures of two or more thereof, more preferably linear $C_{1-6}$-alcohols, yet more preferably $C_{1-4}$-alcohols, even more preferably linear $C_{1-4}$-alcohols, still more preferably methanol, ethanol, n-propanol or isopropanol and most preferably ethanol. Preferred water-alcohol mixtures are mixtures containing 30 to 70% by volume of a $C_{1-6}$-alcohol, more preferably a $C_{1-4}$-alcohol, even more preferably a $C_{1-3}$-alcohol and most preferably ethanol.

It is particularly preferred that component b1) is obtained through an optimized aqueous or hydroalcoholic extraction process, preserving its active constituents such as flavonoids, amino acids, phenolic acids, and polyphenols. These compounds work synergistically to deliver multiple benefits: Flavonoids (e.g., orientin, isoorientin) provide potent antioxidant properties that protect hair from oxidative stress caused by environmental factors like UV exposure and pollution. Amino Acids (e.g., serine, glycine, proline) help repair and fortify the hair structure while enhancing scalp hydration and nourishment. Phenolic Acids and Polyphenols exhibit anti-inflammatory and soothing effects, making the extract suitable for sensitive or irritated scalps, while supporting overall scalp health and reducing dandruff.

*b2) cactus extract:*

Cactus extract (component b2)) is an extract derived from any part of a cactus plant, such as from pads, stems, flowers and/or fruits, within the plant family Cactaceae, renowned for its hydrating and protective properties. Suitable genera of Cactaceae are Opuntia, Cereus, Carnegiea, Pachycereus, Stenocereus, Neobuxbaumia, Pilosocereus, Cephalocereus, Stephanocereus, Armatocereus, Lemaireocereus, Ferocactus, Echinocactus, Thelocactus, *Parodia,* Echinopsis, Gymnocalycium, Mammillaria, Rebutia, Astrophytum, Lophophora, Cylindropuntia, Tephrocactus, Austrocylindropuntia, Maihuenia, Epiphyllum, Schlumbergera, Hatiora, Ariocarpus, Turbinicarpus and Blossfeldia.

Examples for suitable extracting agents are water, alcohols and water-alcohol mixtures. Preferred alcohols are $C_{1-6}$-alcohols or mixtures of two or more thereof, more preferably linear $C_{1-6}$-alcohols, yet more preferably $C_{1-4}$-alcohols, even more preferably linear $C_{1-4}$-alcohols, still more preferably methanol, ethanol, n-propanol or isopropanol and most preferably ethanol. Preferred water-alcohol mixtures are mixtures containing 30 to 50% by volume of a $C_{1-6}$-alcohol, more preferably a $C_{1-4}$-alcohol, even more preferably a $C_{1-3}$-alcohol and most preferably ethanol.

Preferably, component b2) is extracted from the pads, stems, flowers and/or fruits of Opuntia ficus-indica (prickly pear cactus), which is particularly rich in bioactive compounds, or *Cereus Grandiflorius.* Component b2) is produced through advanced water, ethanol, or mixed solvent extraction processes, designed to maximize the preservation of its beneficial constituents. These include: Polysaccharides (e.g., mucilage) provide superior hydration by forming a moisture-retentive barrier on both hair and scalp, which is especially effective in dry or arid conditions. Vitamins (e.g., vitamin E, vitamin C, and B vitamins) deliver powerful antioxidant protection against free radicals, helping to prevent environmental damage and oxidative stress on the hair and scalp. Minerals (e.g., calcium, magnesium, potassium) support overall scalp health and contribute to strengthening hair roots, reducing hair fall. Amino Acids (e.g., proline, taurine) enhance scalp hydration, repair damage, and promote elasticity in hair strands, minimizing brittleness.

*b3) jojoba oil:*

Jojoba oil (component b3)) is preferably obtained from the seeds of Simmondsia chinensis, commonly in the form of a liquid wax ester.

Component b3) preferably comprises primarily long-chain monounsaturated fatty acids (e.g., eicosenoic acid) and long-chain alcohols (e.g., docosanol, tetracosanol). Additionally, it is preferably rich in tocopherols (vitamin E), which provide antioxidant properties, and contains phytosterols that contribute to soothing and protecting the skin.

The production of component b3) (jojoba oil) preferably involves cold pressing the seeds of Simmondsia chinensis, followed by filtration to ensure purity.

*b4) safflower oil:*

Safflower oil (component b4)) is preferably obtained from the seeds of Carthamus tinctorius.

Component b4) predominantly comprises linoleic acid (up to 75%), an omega-6 fatty acid, along with oleic acid and smaller proportions of palmitic and stearic acids. It is preferably enriched with vitamin E (tocopherols) and phytosterols (such as β-sitosterol, campesterol, and stigmasterol), which provide anti-inflammatory and moisturizing benefits.

The production of component b4) (safflower oil) preferably involves cold pressing or solvent extraction methods.

Jojoba oil and safflower oil preferably work synergistically to ensure optimal moisture retention in hair and scalp care. Jojoba oil, a liquid wax ester derived from Simmondsia chinensis, closely mimics the natural sebum of the scalp, creating a lightweight, non-greasy barrier that locks in moisture and prevents dehydration. Its rich content of monounsaturated fatty acids and tocopherols enhances scalp health while smoothing the hair cuticle. Complementing this, safflower oil, derived from Carthamus tinctorius, is high in linoleic acid, an omega-6 fatty acid, which penetrates the hair shaft to restore lipid balance and reinforce the hair's structural integrity. Together, these oils form a dual-action system where jojoba oil seals and protects the hair's outer layer, while safflower oil deeply nourishes and hydrates from within, resulting in soft, resilient, and well-moisturized hair. Their combined action not only prevents moisture loss but also enhances overall scalp health, making this pairing ideal for maintaining hydration in various hair types.

*b5) olive oil:*

Olive oil (component b5)) is preferably obtained from the fruits of Olea europaea.

Component b5) primarily comprises monounsaturated fatty acids, with oleic acid (55-83%) as the major component, along with smaller amounts of linoleic acid, palmitic acid, and stearic acid. It preferably contains phenolic compounds (e.g., hydroxytyrosol and oleuropein), squalene, tocopherols (vitamin E), and phytosterols, which provide antioxidant protection, emollient properties, and conditioning benefits, making it well-suited for applications in hair and skin care.

The production of component b5) (olive oil) preferably involves cold pressing or solvent extraction techniques.

*B6) babassu oil:*

Babassu oil (component b6)) is preferably obtained from the seeds of Attalea speciosa (babassu palm).
Component b6) primarily consists of medium-chain fatty acids, with lauric acid (up to 50%) as the major component, along with myristic acid, palmitic acid, and stearic acid. It also preferably contains small quantities of phytosterols (e.g., β-sitosterol, stigmasterol) and tocopherols (vitamin E), making it a versatile emollient that effectively provides moisture without leaving a greasy residue.
The production of component b6) (babassu oil) preferably involves mechanical pressing or solvent extraction methods.

*b7) sesame seed Oil:*

Sesame seed oil (component b7)) is preferably obtained from the seeds of Sesamum indicum.
Component b7) primarily contains oleic acid (35-50%) and linoleic acid (35-50%), along with smaller amounts of palmitic acid, stearic acid, and arachidic acid. It is distinguished by its antioxidant components, including lignans (such as sesamin, sesamolin, and sesaminol), tocopherols (vitamin E), and phytosterols (e.g., β-sitosterol, campesterol), which enhance its stability and nourishing properties.
The production of component b7) (sesame seed oil) preferably involves cold pressing or solvent extraction processes.

*b8) rice bran oil:*

Rice bran oil (component b8)) is preferably obtained from the bran layer of Oryza sativa (rice).
Component b8) features a balanced fatty acid profile, predominantly oleic acid (up to 50%), linoleic acid (up to 35%), and palmitic acid. It is notably rich in gamma oryzanol, a distinctive antioxidant comprising a mixture of ferulic acid esters of sterols and triterpene alcohols. Additionally, component b8) preferably includes tocopherols and tocotrienols (vitamin E), squalene, and phospholipids, which enhance its antioxidant properties, amplify conditioning effects, and improve smoothing benefits.
The production of component b8) (rice bran oil) preferably involves cold pressing or solvent extraction techniques.

*b9) evening primrose oil:*

Evening primrose oil (component b9)) is preferably obtained from the seeds of Oenothera biennis.
Component b9) is characterized by a high concentration of gamma-linolenic acid (GLA), an omega-6 fatty acid, along with significant amounts of linoleic acid, oleic acid, and palmitic acid. It preferably also contains phytosterols (e.g., β-sitosterol) and tocopherols (vitamin E), which contribute to its anti-inflammatory and moisturizing properties.
The production of component b9) (evening primrose oil) is preferably achieved through cold pressing.
In a preferred embodiment the botanical mixture (BM) comprises

1.0 to 10.0 % by weight of component b1),
1.0 to 10.0 % by weight of component b2),
10.0 to 30 % by weight of component b3),
10.0 to 30 % by weight of component b4),
5.0 to 15 % by weight of component b5),
5.0 to 15 % by weight of component b6),
5.0 to 15 % by weight of component b7),
5.0 to 15 % by weight of component b8),
5.0 to 15 % by weight of component b9),

wherein the % by weight values are based on the total weight of the components b1), b2), b3), b4), b5), b6), b7), b8), and b9) comprised in the botanical mixture (BM), wherein the sum of the % by weight values of the components b1), b2), b3), b4), b5), b6), b7), b8), and b9) is 100 % by weight.
Preferably the % by weight values are based on the total weight of the botanical mixture (BM), wherein the sum of the % by weight values of the components b1), b2), b3), b4), b5), b6), b7), b8), and b9) is less than or equal to 100 % by weight. If the sum of components b1) to b9) is 100 % by weight of the botanical mixture (BM), the botanical mixture (BM) consists of the components b1), b2), b3), b4), b5), b6), b7), b8), and b9), whereas in the case that the sum of components b1) to b9) is less than 100 % by weight of the botanical mixture (BM), the botanical mixture (BM) comprises the components b1), b2), b3), b4), b5), b6), b7), b8), and b9) and one or more further components.

Hair cosmetic product

It was surprisingly found that the botanical mixture can be advantageously used in a hair cosmetic product.

In a preferred embodiment of the present invention the hair cosmetic product comprises 0.05 to 5 % by weight, more preferred 0.05 to 2.0 % by weight, and particularly preferred 0.05 to 1.6 % by weight of the botanical mixture (BM) based on the total weight of the hair cosmetic product.

In another preferred embodiment of the present invention the hair cosmetic product comprises at least one cosmetically acceptable aqueous or aqueous-organic medium.

The term "at least one cosmetically acceptable aqueous or aqueous-organic medium" means exactly one cosmetically acceptable aqueous or aqueous-organic medium and, also a mixture of two or more different cosmetically acceptable aqueous or aqueous-organic mediums.

In a preferred embodiment of the present invention the hair cosmetic product comprises the cosmetically acceptable aqueous or aqueous-organic medium in an amount in the range of 50 to 90 % by weight based on the total weight of the hair cosmetic product.

The cosmetically acceptable aqueous medium consists preferably of water. In another embodiment the cosmetically acceptable aqueous-organic medium consists preferably of water and a water miscible organic solvent such as an alcohol.

In another embodiment the cosmetically acceptable aqueous-organic medium comprises preferably water and a monoalcohol such as ethanol and isopropanol and/or a polyalcohol such as propylene glycol, hexylene glycol, glycerin, and propane diol.

In another embodiment the cosmetically acceptable aqueous-organic medium comprises the organic medium as a major component.

In a preferred embodiment the hair cosmetic product comprises at least one fatty substance.

The term "at least one fatty substance" means exactly one fatty substance and, also a mixture of two or more different fatty substances.

In a preferred embodiment of the present invention the hair cosmetic product comprises the fatty substance in an amount in the range of 5 to 25 % by weight based on the total weight of the hair cosmetic product.

The at least fatty substance is different form the 9 botanical components comprised in the botanical mixture (BM).

In one embodiment of the presently claimed invention, the hair cosmetic product comprises preferably at least one fatty substance that does not contain any free carboxylic acid groups, i.e. free of -C(=O)-OH groups and -C(=O)-O$^-$ groups. The term "fatty substance" means an organic compound that is insoluble in water at room temperature (25°C) and at atmospheric pressure ($\leq$ 15 mg/L water, preferably $\leq$ 5 mg/L water). In addition, under the same temperature and pressure conditions, the fatty substances are soluble in organic solvents such as chloroform, ethanol, or benzene.

In a preferred embodiment of the presently claimed invention, the fatty substances are selected from the group consisting of liquid hydrocarbons, non-silicone oils of animal, plant, mineral or synthetic origin, fatty alcohols, fatty acid esters and/or fatty alcohol esters, non-silicone waxes, and silicones or mixtures thereof.

More particularly, the liquid hydrocarbons are selected from the group consisting of:

-    linear or branched, optionally cyclic, C6-C16 alkanes such as hexane, undecane, dodecane, tridecane, and isoparaffins, such as isohexadecane, isododecane and isodecane,

-    linear or branched hydrocarbons of mineral, animal or synthetic origin with more than 16 carbon atoms, such as volatile or non-volatile liquid paraffins and derivatives thereof, petroleum jelly, liquid petroleum jelly, polydecenes, hydrogenated polyisobutene such as Parleam$^®$, and squalane.

In a preferred embodiment of the presently claimed invention, the liquid hydrocarbons are selected from the group consisting of volatile or non-volatile liquid paraffins, and liquid petroleum jelly.

In one embodiment of the presently claimed invention the hair cosmetic product, a creamy carrier for the composition comprises preferably at least one fatty alcohol with 10 to 24 carbon atoms; and/or one or more of at least one diester of formula: $R^1$-CO-O-(CH2-CH2-O)$_n$-CO-$R^2$, where n is 1, 2 or 3, and $R^1$ and $R^2$ are the same or different alkyl radicals with 12 to 20 carbon atoms; and/or one or more of glycerine fatty acid ester with 10 to 24 carbon atoms; and/or one or more of non-ionic and/or anionic and/or ampholytic emulsifiers.

In preferred embodiment the hair cosmetic product comprises at least one surfactant.

The term "at least one surfactant" means exactly one surfactant and, also a mixture of two or more different surfactants.

In a preferred embodiment of the present invention the hair cosmetic product comprises the surfactant in an amount in the range of 5 to 25 % by weight based on the total weight of the hair cosmetic product.

The surfactant(s) suitable for the hair cosmetic products include(s) anionic, cationic, amphoteric and non-ionic surfactants.

The anionic surfactants may be selected from the group consisting of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, alpha-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates; N-acyltaurates; and mixtures thereof. The alkyl or acyl radical of all of these various compounds, for example, comprises from 8 to 24 carbon atoms, and the aryl radical, for example, is chosen from phenyl and benzyl groups. Weakly anionic surfactants can also be used, such as alkyl-D-galactosiduronic acids and their salts, as well as polyoxyalkylenated (C6-C24) alkyl ether carboxylic acids, polyoxyalkylenated (C6-C24) alkylaryl ether carboxylic acids, polyoxyalkylenated (C6-C24) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, can be also used.

Suitable anionic surfactant(s) may comprise at least one anionic functional groups at their head selected from sulfate, sulfonate, and phosphate and carboxylates. In order to avoid any overlap with an active ingredient combination of the present invention, anionic surfactant(s) for use in the compositions according to the present invention exclude fatty acids or salts thereof.

Suitable alkyl sulfates include ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate. Further suitable anionic surfactants may include docusate (dioctyl sodium sulfosuccinate), alkyl-aryl ether phosphate, alkyl ether phosphate, sodium lauroyl sarcosinate, ammonium laureth sulfate, disodium lauryl sulfosuccinate, and sodium lauryl sulphoacetate.

Preferred anionic surfactants may be selected from the group consisting of sodium laurylethersulfate, sodium laurethethersulfate, sodium dodecyl sulfate, ammonium laurethethersulfat, ammonium dodecyl sulfate, alkylbenzenesulfonate, and combinations thereof.

Non-ionic surfactants are a versatile and widely used class of surfactants in hair cosmetics due to their mildness, compatibility with other ingredients, and ability to stabilize emulsions. These surfactants do not ionize in water, making them less sensitive to pH and the presence of salts, which ensures consistent performance in various formulations. Non-ionic surfactants suitable for use in hair cosmetics can be selected from a broad range of compounds, including polyoxyethylene ethers of fatty alcohols, fatty acids, fatty esters, and alkylphenols.

Particularly preferred non-ionic surfactants include polyoxyethylated fatty alcohols, such as ceteareth-n, where n typically ranges from 2 to 100, with a preferred range of 10 to 30 for optimal balance of hydrophilicity and lipophilicity. These surfactants are effective in providing conditioning, emolliency, and mild cleansing properties. Lanolin alcohol and its derivatives are also popular for their moisturizing and emollient benefits in hair care formulations. Additionally, polyoxyethylated oils, such as polyoxyethylated castor oil and hydrogenated castor oil, are highly valued for their ability to enhance formulation stability and improve the distribution of active ingredients. Examples include PEG-35 Castor Oil and PEG-40 Hydrogenated Castor Oil, which are particularly beneficial in leave-in treatments and hair masks due to their moisturizing and softening properties.

Other suitable non-ionic surfactants include sorbitan esters, such as polysorbates (e.g., Polysorbate 20, 40, 60, or 80), which are effective emulsifiers and solubilizers, often used to incorporate essential oils or fragrances into aqueous formulations. Alkyl polyglucosides, derived from sugar and fatty alcohols, such as decyl glucoside or lauryl glucoside, are mild, biodegradable options ideal for sulfate-free or sensitive scalp formulations. Fatty acid esters, such as glyceryl stearate and PEG-100 stearate, are also commonly used for their dual roles as emulsifiers and skin conditioners.

For more specialized functions, ethoxylated silicones, such as dimethicone copolyol, are employed for their superior conditioning and detangling effects, particularly in rinse-off conditioners and styling products. Non-ionic surfactants can also include amphiphilic molecules, such as alkoxylated alcohols and ethers, which enhance solubility and improve formulation texture.

These non-ionic surfactants, as detailed in references like "Handbook of Surfactants" by M. R. Porter, contribute significantly to the performance and aesthetic properties of hair cosmetic formulations, enabling the creation of products that are gentle, effective, and enjoyable to use.

In a preferred embodiment the hair cosmetic product according to the invention has a pH value in the range of 5.5 to 6.5.

The hair cosmetic product may optionally comprise auxiliary components. The auxiliary component covers a broad spectrum of substances. The auxiliary components are different from the 9 botanical components.

The hair cosmetic product may comprise an oil component covering natural essential oils and modified oils (different from the 9 botanical components) and mineral oils. Modified oils include in particular hydrogenated oils and oil constituents. The mineral component covers minerals in water-soluble or alcohol-soluble form.

According to embodiments, hair cosmetic product according to the present invention further may comprise auxiliary components comprising in particular vasodilators, blood circulation stimulators, antioxidants, vitamins, provitamins. If present, the amount of the auxiliary component or combinations thereof will be in the range of from 0.0010 to 5.0, in particular from 0.010 to 3.0 percent-by-weight, based on the weight of the hair cosmetic product.

The auxiliary component may comprise, for example, one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol (provitamin B5), omega-3 fatty acids, omega-6 fatty acids, folates, cannabidiol, amino acids, or combinations thereof.

According to embodiments, the auxiliary component includes a vasodilator selected from tocopherols (alpha-, beta-, gamma- or delta-tocopherol), in particular alpha-tocopherol, tocopherol esters (including, for example, tocopherol acetate, tocopheryl succinate, tocopherylnicotinate, tocopherylpoly(oxyethylene)-succinate), nicotinic acid, nicotinic amides, nicotinic esters, camphor; antioxidants such as vitamin C, vitamin E, polyphenols (e.g., green tea extract, grape seed extract, resveratrol), coenzyme Q10, carotenoids (e.g., beta-carotene, lycopene, astaxanthin), ferulic acid, glutathione, flavonoids (e.g., quercetin, rutin, catechins), squalene, alpha-lipoic acid (ALA), niacinamide (vitamin B3), tannins (e.g., witch hazel or chestnut extracts), hydroxytyrosol (from olive extract), silk amino acids, and caffeine; an inhibitor of testosterone-5-alpha-reductase such as the omega-6 fatty acid arachidonic acid; a substance providing a benefit to hair health such as nicotinic amide (niacinamide); or a combination thereof. According to embodiments, the auxiliary component includes tocopherol or tocopheryl acetate (vitamin E acetate), and/or niacinamide. Suitable vasodilatory plant extracts include burned or distilled nettle extract (Urtica Diocia), roast chestnut extract (Aesculus Hippocastanum), Arnica extract (Arnica Montana), or hemp extract.

The hair cosmetic product according to the present invention optionally may comprise cosmetically acceptable additives According to embodiments, the cosmetically acceptable additives may be selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents. The cosmetically acceptable additives may be used in any combinations, as desired or required.

Suitable cosmetically acceptable additives not specifically described below are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific additives to achieve a particular purpose or multipurpose. A few of these additives are discussed below, whose invention is of course non-exhaustive.

Suitable amount ranges for perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents are indicated for additives discussed below. These cosmetically acceptable additives may be present in a total amount of up to 20.0, in particular up to 15.0 percent-by-weight, based on the weight of the hair cosmetic product. Typically, the total amount of these cosmetically acceptable additives may be within a range of from 0.010 to 10.0, conveniently within a range of from 0.010 to 7.0 percent-by-weight, based on the weight of the hair cosmetic product. For example, the total amount of these cosmetically acceptable additives may be within a range of from 0.010 to 5.0, conveniently within a range of from 0.010 to 3.0 percent-by-weight, based on the weight of the hair cosmetic product.

The hair cosmetic product according to the present invention may comprise a perfume or fragrance. For example, the hair cosmetic product may comprise from 0.0010 to 1.50, in particular 0.010-0.50 percent-by-weight perfume, based on the weight of the hair cosmetic product. Perfume can provide an enhanced user experience by making the composition smell pleasant and/or invoke emotions, such as relaxing or exciting smells.

Alternatively, the hair cosmetic product according to the present invention may be substantially free of perfume and/or fragrance. Some consumers prefer perfume-free compositions. The perfume may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume may comprise one or more scents selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, $\alpha$-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-$\alpha$-naphthyl ketone, methylphenylcarbinyl acetate, mugetanol, $\gamma$-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, $\gamma$-undecalactone, undecenal, vanillin, veloutone, verdox, and mixtures thereof.

The hair cosmetic product used in accordance with the present invention may comprise an opacifier, which sometimes is denoted as well by the term "turbidity-inducing agent". For example, the hair cosmetic product may comprise from 0.10 to 5.0, in particular 0.50-2.0 percent-by-weight opacifier, based on the weight of the hair cosmetic product. Suitable opacifiers include mineral pigments such as, for example, titanium dioxide, alumina, calcium carbonate.

The hair cosmetic product according to the present invention may comprise a preservative or mixture of preservatives. The hair cosmetic product may comprise the preservative(s) in an amount of up to 1.0, for example from 0.0010 to 1.0, in particular from 0.010 to 0.50 percent-by-weight, based on the weight of the hair cosmetic product. Cosmetically acceptable preservatives include organic acids such as para-hydroxybenzoic acid; organic acid salts such as sodium benzoate; compounds such as benzyl alcohol, phenoxyethanol, 1,3-bis(hydroxymethyl)-5,5-dimethylimidazolidine-2,4-dione; and mixtures thereof.

The hair cosmetic product according with the present invention may comprise a pH modifier and/or buffering agent, in a sufficient amount to effectively adjust the pH of the composition. For example, the hair cosmetic product may comprise up to 1.0, for example from 0.000010 to 1.0, in particular 0.0010-0.50 percent-by-weight pH modifiers, based on the weight of the hair cosmetic product. Suitable pH modifiers and/or buffering agents for use herein include, but are not limited to: ammonia, alkanolamines such as monoethanolamine, diethanolamine, triethanolamine, monopropanolamine, dipropanolamine, tripropanolamine, tripropanolamine, 2-amino-2-methyl-1-propanol, and 2-amino-2-hydroxymethyl-1,3,-propandiol and guanidium salts, alkali metal and ammonium hydroxides and carbonates, such as sodium hydroxide, sodium silicate, sodium meta silicate and ammonium carbonate, and acids such as organic and inorganic acids, for example ascorbic acid, citric acid, acetic acid or tartaric acid, phosphoric acid, hydrochloric acid, and mixtures thereof.

Preferred alkaline pH modifiers include ammonia, ethanolamines, and alkali metal hydroxides, in particular sodium hydroxide. Preferred acidic pH modifiers include hydrochloric acid, ascorbic acid, or citric acid.

The according to the present invention may further comprise one or more chelators (also known as "chelating agent", "sequestering agent", "sequestrant", or chelants) in an amount sufficient to reduce the amount of metals available to interact with formulation components. Chelators are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996). Typically, the compositions may comprise one or more chelators in a total amount of from 0.0010 to 1.0, in particular from 0.010 to 0.50 percent-by-weight, based on the weight of the.

The one or more chelators may be selected from the group consisting of carboxylic acids (such as aminocarboxylic acids), phosphonic acids (such as aminophosphonic acids), polyphosphoric acids (such as linear polyphosphoric acids), their salts thereof, and mixtures thereof. By "salts thereof", it is meant - in the context of chelators - all salts comprising the same functional structure as the chelators they are referring to and including alkali metal salts, alkaline earth salts, ammonium salts, substituted ammonium salts, and mixtures thereof; alternatively sodium salts, potassium salts, calcium salts, magnesium salts, ammonium salts, and mixtures thereof; alternatively monoethanolammonium salts, diethanolammonium salts, triethanolammonium salts, and mixtures thereof.

The one or more chelators may be one or more aminocarboxylic acid chelators comprising one or more carboxylic acid moieties (-COOH) and one or more nitrogen atoms. The one or more aminocarboxylic acid chelators may be selected from the group consisting of diethylenetriamine pentaacetic acid (DTPA), ethylenediamine disuccinic acid (EDDS), ethylenediamine-N,N'-diglutaric acid (EDDG), 2-hydroxypropylenediamine-N-N'-disuccinic acid (HPDDS), glycinamide-N,N'-disuccinic acid (GADS), ethylenediamine-N-N'-diglutaric acid (EDDG), 2-hydroxypropylenediamine-N-N'-disuccinic acid (HPDDS), ethylenediaminetetraacetic acid (EDTA), ethylenedicysteic acid (EDC), ethylenediamine-N-N'-bis(ortho-hydroxyphenyl acetic acid) (EDDHA), diaminoalkyldi(sulfosuccinic acids) (DDS), N,N'-bis(2-hydroxybenzyl)ethylenediamine-N,N'-diacetic acid (HBED), their salts thereof, and mixtures thereof. Conveniently, a chelator used in compositions according to the present invention, if any, may be EDTA.

The hair cosmetic product according to the present invention may comprise viscosity modifiers, referred to in the art as well as "thickening agents". If present, such viscosity modifiers typicall wil be used in the hair cosmetic product in an amount of from 0.010 to 3.0, in particular from 0.50 to 1.0 percent-by-weight, based on the weight of the hair cosmetic product. Suitable cosmetically acceptable viscosity modifiers include non-ionic polymers, for example polysaccharides such as cellulose, cellulose gum or xanthan gum, and non-ionic viscosity modifiers based on polyether-1 such as the commercially available viscosity modifiers Pure Thix 1442.

The hair cosmetic product according to the present invention may further comprise care materials, such as hair-care providing or skin-care-providing plant or vegetable extracts (different from the 9 botanical components), cationic polymers or cationic surfactants. Typical amounts of care materials in the compositions according to the present invention will be in the range of from 0.010 to 5.0, in particular 0.050-2.0 percent-by-weight, based on the weight of the hair cosmetic product.

Additional cosmetically acceptable additives, which may be present in the according to the present invention include:

- physiologically compatible silicone derivative compounds, such as volatile or non-volatile silicone or high molecular weight siloxane polymers, such as for example Quaternium-80, typically in an amount of from 0.05 to 20 percent-by-weight, in particular from 0.10 to 5.0 percent-by-weight, based on the weight of the composition
- light protecting agents, such as for example octylmethoxy cinnamate
- anti-flaking agents, typically in an amount of about 0.01 to 2 percent-by-weight
- hair luster-imparting agents, such as for example phenyltrimethicone,
- combability improving agents, such as for example cationic surfactants such as Behentrimonium Chloride, or cationic polymers such as Polyquaternium-10
- defatting agents
- conditioning agents
- moisturizing agents, such as for example sodium lactate, sodium PCA, glycine, fructose, urea, niacinamide, inositol
- cooling agents, such as for example menthol, eucalptol, geraniol, linalool, isopulegol, cubedol, p-mentane-3,8-diols, hydroxy-citronellal as well as esters and derivatives thereof; ketones such as menthone and carvone; carboxamides, or evaporative cooling agents

The cosmetically acceptable additives may be used in the hair cosmetic product according to the present invention in any combinations, as desired or required.

In one embodiment the hair cosmetic product according to the present invention may be essentially free of, or be free of certain substances or classes of chemical compounds.

In an especially preferred embodiments the hair cosmetic product according to the present invention may be essentially free of silicones. More preferred the hair cosmetic product according to the present invention may be free of silicones. According to particular embodiments, the concentration of silicones may be less than 5 ppm by weight based on the weight of the hair cosmetic product.

In an especially preferred embodiment the hair cosmetic product is selected from the group consisting of shampoos, rinse-off conditioners, hair masks, hair treatments, leave-in conditioners, hair serums, hair oils, hair creams, hair lotions, styling gels, styling creams, mousses, foams, heat protectant sprays, hair sprays, dry shampoos, hair pomades, hair waxes, hair butters, scalp treatments, scalp serums, anti-frizz sprays, anti-frizz creams, detangling sprays, hair perfumes, hair relaxers, hair straightening creams, hair dyes, hair coloring treatments, hair growth serums, curl enhancers, curl creams, split-end repair treatments, hair setting sprays, texturizing sprays and powders.

In a particularly preferred embodiment, the hair cosmetic product is a shampoo or a conditioner.

The hair cosmetic product according to the present invention are preferably topically applied on at least part of the scalp region, for at least one minute, preferably for at least 1.5 minutes and more preferably for at least 2 minutes.

The hair cosmetic product may be left on the scalp hair (leave-in) or may be removed after applying the hair cosmetic product to the scalp hair (rinse-off)

Another aspect of the present invention is a method for treating hair comprising applying the botanical mixture (BM) according to the invention or the hair cosmetic product according to the invention to a person's scalp hair.

Another aspect of the present invention it the use of the botanical mixture (BM) according to the invention or the hair cosmetic product according to the invention for treating human hair.

Yet another aspect of the present invention is the use of the botanical mixture (BM) according to invention or the hair cosmetic product according to the invention for nourishing and/or strengthening human hair.

Yet another aspect of the present invention is the use of the botanical mixture (BM) according to the invention or the hair cosmetic product according to the invention for the reduction of the combing force of human hair.

The combing force is preferably measured in accordance with the method proposed by Garcia & Diaz (JSCC, 27, (1976) 379-398 - Combability Measurements on Hair).

**Embodiments**

In the following, there is provided a list of embodiments to further illustrate the present invention without intending to limit the invention to the specific embodiments listed below.

   1. Botanical mixture (BM) comprising the following 9 botanical components

      b1) bamboo extract,
      b2) cactus extract,
      b3) jojoba oil,
      b4) safflower oil,
      b5) olive oil,
      b6) babassu oil,
      b7) sesame seed oil,
      b8) rice bran oil, and
      b9) evening primrose oil.

   2. The botanical mixture (BM) according to embodiment 1, wherein the botanical mixture (BM) comprises

      1 to 10 % by weight of component b1),
      1 to 10 % by weight of component b2),
      10 to 30 % by weight of component b3),
      10 to 30% by weight of component b4),
      5 to 15 % by weight of component b5),
      5 to 15 % by weight of component b6),
      5 to 15 % by weight of component b7),
      5 to 15 % by weight of component b8),
      5 to 15 % by weight of component b9),

   wherein the % by weight values are based on the total weight of the botanical mixture (BM), wherein the sum of components b1) to b9) is less than or equal to 100 % by weight of the botanical mixture (BM).

   3. A hair cosmetic product comprising the botanical mixture (BM) according to embodiment 1 or 2.

   4. The hair cosmetic product according to embodiment 3, wherein the hair cosmetic product comprises 0.05 to 5.0 % by weight of the botanical mixture (BM) based on the total weight of the hair cosmetic product.

   5. The hair cosmetic product according to embodiment 3 or 4, wherein the hair cosmetic product comprises at least one cosmetically acceptable aqueous or aqueous-organic medium.

   6. The hair cosmetic product according to embodiment 5, wherein the hair cosmetic product comprises the at least one cosmetically acceptable aqueous or aqueous-organic medium in an amount in the range of 50 to 90 % by weight based on the total weight of the hair cosmetic product.

   7. The hair cosmetic product according to any one of embodiments 3 to 6, wherein the hair cosmetic product comprises at least one fatty substance being different form the 9 botanical components comprised in the botanical mixture (BM).

   8. The hair cosmetic product according to embodiment 7, wherein the hair cosmetic product comprises the at least one fatty substance in an amount in the range of 5 to 25 % by weight based on the total weight of the hair cosmetic product.

   9. The hair cosmetic product according to any one of embodiments 3 to 8, wherein the hair cosmetic product comprises at least one surfactant.

   10. The hair cosmetic product according to embodiment 9, wherein the hair cosmetic product comprises the at least one surfactant in an amount in the range of 5 to 25 % by weight based on the total weight of the hair cosmetic product.

EP 4 759 375 A1

11. The hair cosmetic product according to any one of embodiments 3 to 10, wherein the hair cosmetic product has a pH value in the range of 5.5 to 6.5.

12. The hair cosmetic product according to any one of embodiments 3 to 11, wherein the hair cosmetic product is selected from the group consisting of shampoos, rinse-off conditioners, hair masks, hair treatments, leave-in conditioners, hair serums, hair oils, hair creams, hair lotions, styling gels, styling creams, mousses, foams, heat protectant sprays, hair sprays, dry shampoos, hair pomades, hair waxes, hair butters, scalp treatments, scalp serums, anti-frizz sprays, anti-frizz creams, detangling sprays, hair perfumes, hair relaxers, hair straightening creams, hair dyes, hair coloring treatments, hair growth serums, curl enhancers, curl creams, split-end repair treatments, hair setting sprays, texturizing sprays and powders.

13. The hair cosmetic product according to any one of embodiments 3 to 12, wherein the hair cosmetic product is a shampoo, a conditioner, a serum or a hair mask comprising at least one cosmetically acceptable aqueous or aqueous-organic medium, at least one fatty substance, and at least one surfactant.

14. The hair cosmetic product according to any one of embodiments 3 to 13, wherein the hair cosmetic product is a shampoo comprising at least one cosmetically acceptable aqueous or aqueous-organic medium, at least one fatty substance, and at least one surfactant.

15. The hair cosmetic product according to any one of embodiments 3 to 13, wherein the hair cosmetic product is a conditioner comprising at least one cosmetically acceptable aqueous or aqueous-organic medium, at least one fatty substance, and at least one surfactant.

16. The hair cosmetic product according to any one of embodiments 3 to 13, wherein the hair cosmetic product is a serum at least one cosmetically acceptable aqueous or aqueous-organic medium, at least one fatty substance, and at least one surfactant.

17. The hair cosmetic product according to any one of embodiments 3 to 13, wherein the hair cosmetic product is a hair mask comprising at least one cosmetically acceptable aqueous or aqueous-organic medium, at least one fatty substance, and at least one surfactant.

18. The hair cosmetic product according to any one of embodiments 3 to 12, wherein the hair cosmetic product is a hair oil comprising the botanical mixture (BM) according to embodiment 1 or 2.

19. The hair oil according to embodiment 18, wherein the hair oil comprises at least one emollient oil, different from the botanical components b1), b2), b3), b4), b5), b6), b7), b8), and b9).

20. The hair oil according to embodiment 18 or 19, wherein the hair oil comprises at least one conditioning agent.

21. The hair oil according to any one of embodiments 18 to 20, wherein the hair oil comprises at least one of the following ingredients of antioxidants, vitamins, UV-filters, fragrances and/or preservatives.

22. The hair oil according to any one of embodiments 18 to 21, wherein the hair oil comprises tocopherol, and/or panthenol.

23. The hair oil according to any one of embodiments 20 to 22, wherein the at least one conditioning agent is at least one selected from the group consisting of dimethicone, cyclopentasiloxane, behentrimonium chloride, cetrimonium chloride, polyquaternium 6, and polyquaternium 10.

24. The hair oil according to any one of embodiments 19 to 23, wherein the emollient is at least one emollient selected from the group consisting of argan oil, coconut oil, almond oil, squalane, squalene, ethyl hexyl palmitate, cetyl ethylhexaloate, and octyldodecanol.

25. The hair cosmetic product according to any one of embodiments 3 to 13, wherein the hair cosmetic product comprises one more or all of the following components

a) at least one C3-C13 saturated or unsaturated dicarboxylic acid, which is unsubstituted or substituted with one hydroxyl group,

b) at least one C10-C30 saturated or unsaturated monocarboxylic,

c) at least one C10-C40 saturated or unsaturated hydrocarbon,

d) at least one fatty acid ester of a C10-C30 saturated or unsaturated monocarboxylic acid and a C3-C6 mono-ol, diol or triol.

26. The hair cosmetic product according to embodiment 25, wherein component a) is at least one C3-C6 saturated or unsaturated dicarboxylic acid, the saturated dicarboxylic acid being optionally substituted with one hydroxyl group.

27. The hair cosmetic product according to embodiment 25 or 26, wherein the component a) is selected from the group consisting of malonic acid, succinic acid, glutaric acid, adipic acid, malic acid, hydroxysuccinic acid, hydroxyglutaric acid, fumaric acid, maleic acid, itaconic acid, glutaconic acid, salts thereof, and any combination thereof.

28. The hair cosmetic product according to any one of embodiments 25 to 27, wherein component b) is at least one C16-C22 saturated or unsaturated monocarboxylic acid.

29. The hair cosmetic product according to any one of embodiments 25 to 28, wherein component d) is at least one fatty acid ester of a C16-C22 saturated or unsaturated monocarboxylic acid and a C3-C6 triol.

30. The hair cosmetic product according to any one of embodiments 25 to 29, wherein component c) is at least one C26-C40 saturated or unsaturated hydrocarbon.

31. The hair cosmetic product according to any one of embodiments 25 to 30, wherein

component a) is at least one selected from the group consisting of malic acid and hydroxyglutaric acid,
component b) is at least one selected from the group consisting of oleic acid and palmitoleic acid,
component c) is at least one selected from the group consisting of squalane and squalene,
component d) is at least one selected from the group consisting of glyceryl monooleate and glyceryl mono-palmitoleate.

32. The hair cosmetic product according to any one of embodiments 3 to 31, wherein the hair cosmetic product comprises less than 5ppm by weight of a silicon containing component

33. The hair cosmetic product according to any one of embodiments 3 to 32, wherein the hair cosmetic product comprises does not contain any silicon containing component(s).

34. A method for treating hair comprising applying botanical mixture (BM) according to embodiment 1 or 2 or the hair cosmetic product according to any one of embodiments 3 to 33 to a person's scalp hair.

35. Use of the botanical mixture (BM) according to embodiment 1 or 2 or the hair cosmetic product according to any one of embodiments 3 to 33 for treating human hair.

36. Use of the botanical mixture (BM) according to embodiment 1 or 2 or the hair cosmetic product according to any one of embodiments 3 to 33 for nourishing and/or strengthening human hair.

37. Use of the botanical mixture (BM) according to embodiment or 2 or the hair cosmetic product according to any one of claims 3 to 33 for the reduction of combing force of human hair.

**EXAMPLES**

The following examples illustrate the formulations and performance results of the botanical mixture (BM) comprised in a hair cosmetic product according to the presently claimed invention. These examples are not intended to be inclusive of all aspects of the subject matter disclosed herein, but rather to illustrate representative compositions and results. These examples are not intended to exclude equivalents and variations of the presently claimed invention, which are apparent to one skilled in the art.

The following hair cosmetic products were tested.

HT1 is a hair cosmetic product of the state of the art (not according to the invention).

HT2 is hair cosmetic products according to the invention.

The components contained in the hair cosmetic products HT1, and HT2 are given in the table below.

| Raw Material Name | HT1 | HT2 |
|---|---|---|
| Component b7) Sesame Seed Oil | - | 0.100000 % |
| Components b1), b2) Cereus G. (Cactus) FE Bambusa Vulgaris (Bamboo) LSE | - | 0.400000 % |
| Component b8) Oryza Sativa (Rice) Bran Oil | - | 0.100000 % |
| Component b5) Olea Europaea (Olive) Fruit Oil, refined | - | 0.100000 % |
| Histidine, USP | 0.015000 % | 0.015000 % |
| Quaternium-80, 50% in Propylene Glycol | 0.50000 % | 0.50000 % |
| Sodium Benzoate | 0.50000 % | 0.50000 % |
| Water Purified, USP, JSQI | q.s. | q.s. |
| Cetyl Alcohol 95% | 1.671000 % | 1.671400 % |
| Stearyl Alcohol 97% | 4.179000 % | 4.178600 % |
| Panthenol 50%, stabilized | 1.0000 % | 1.0000 % |
| Component b3) Simmondsia Chinensis (Jojoba) Seed Oil | - | 0.100000 % |
| Component b6) Orbignya Oleifera Seed Oil | - | 0.100000 % |
| Component b4) CARTHAMUS TINCTORIUS(SAFFLOWER) SEED OIL | - | 0.100000 % |
| Component b9) Oenothera Biennis (Evening Primerose) Oil | - | 0.100000 % |
| Behentrimonium Chloride in Isopropyl Alcohol | 2.848100 % | 2.848100 % |
| Citric Acid Anhydrous | 0.100000 % | 0.100000 % |
| NECTARIA LITHOPS-C 01 | 0 % | 0 % |
| BOTANICAL MOISTURE 120451 | 0.50000 % | 0.50000 % |
| TOTALS: | 100.000000 % | 100.000000 % |

The hair cosmetic products HT1, and HT2 were applied to tresses. As tresses European Medium Brown, 6% Bleached hair tresses, (3.0g, 8" length, 1" wide) were used for each treatment group.

In each treatment group 8 tresses are used. All tresses used in the treatment groups are treated with a non-conditioning shampoo comprising 15 wt.-% of Sodium laureth sulfate (SLES) prior to the application of hair cosmetic products.

Treatment group (TG0):

TG0 is the control group, no hair cosmetic product is used. On damp hair (tresses) the non-conditioning shampoo comprising 15 wt.-% of sodium laureth sulfate (SLES) is applied. Dose at 0.1 g per g of (dry) hair, massage 30 sec, rinse 30 sec. Two cycles, 1 cycle and 2 cycle were carried out.

Treatment group (TG1):

In TG1 the hair cosmetic product HT1 (not according to the invention) is used. On damp hair (tresses) the non-conditioning shampoo comprising 15 wt.-% of sodium laureth sulfate (SLES) is applied. Dose at 0.1 g per g of (dry) hair, massage 30 sec, rinse 30 sec. Subsequently the hair cosmetic product HT1 is applied to damp hair (dresses). Dose at 0.15g per g of (dry) hair. Apply for 30 sec, rinse 30 sec. Two cycles, 1 cycle and 2 cycle were carried out.

Treatment group (TG2):

In TG2 the hair cosmetic product HT2 (according to the invention) is used. On damp hair (tresses) the non-conditioning shampoo comprising 15 wt.-% of sodium laureth sulfate (SLES) is applied. Dose at 0.1 g per g of (dry) hair, massage 30 sec, rinse 30 sec. Subsequently the hair cosmetic product HT2 is applied to damp hair (dresses). Dose at 0.15g per g of (dry) hair. Apply for 30 sec, rinse 30 sec. Two cycles, 1 cycle and 2 cycle were carried out.

The primary technical function of hair cosmetic products is to lubricate the hair surface; and, in doing so, facilitate manageability and provide detangling benefits and lower combing friction. A common and highly consumer-relevant approach for measuring this lubrication involves an instrumental combing experiment. Testing involves use of an Instron tensile tester to measure frictional forces while a hair tress is pulled through a comb.

The combing force was measured on wet tresses (wet combing force) and on dry tresses (dry combing force). The combing force was measured after cycle 1 and after cycle 2.

The wet and dry combing force was measured in accordance with the method proposed by Garcia & Diaz (JSCC, 27, (1976) 379-398 - Combability Measurements on Hair). Combing experiments were performed in the wet state and in the dry state after treatment. Six combing strokes are performed per tress, while eight replicate hair tresses are used per treatment group to ensure statistical relevance.

Eight (8) tresses (each 3.0g, 8in in length) were used per treatment group. Six (6) measurements were taken per tress using an Instron tensile tester to evaluate product performance.

The wet combing force was measured directly after the treatment. For measuring the dry combing force, the dresses were treated as outlined above and allowed to equilibrate overnight in a controlled temperature and humidity room (60% RH). Dry combing measurements were performed next morning

The reduction of combing force is calculated according to the equation shown below:

$$\% \text{ reduction combing force} = \left(1 - \frac{\text{Combing force of treatment group (TG1 or TG2)}}{\text{Combing force of control croup TG0}}\right) \times 100$$

The values for the % reduction in combing force are shown in the below tables.

| Reduction in wet combing forces compared to control (TGO) after 1 cycle | |
|---|---|
| Treatment Group | Percent reduction in combing force |
| TG1 | 88.8 |
| TG2 | 86.4 |

| Reduction in wet combing forces compared to control (TGO) after 2 cycle | |
|---|---|
| Treatment Group | Percent reduction in combing force |
| TG1 | 89.1 |
| TG2 | 87.0 |

| Reduction in dry combing forces compared to control (TGO) after 1 cycle | |
|---|---|
| Treatment Group | Percent reduction in combing force |
| TG1 | 71.4 |
| TG2 | 68.3 |

| Reduction in dry combing forces compared to control (TGO) after 2 cycle | |
|---|---|
| Treatment Group | Percent reduction in combing force |
| TG1 | 60.7 |
| TG2 | 56.7 |

The experimental data prove that hair cosmetic products comprising the botanical mixture (BM) according to the present invention led to a significant reduction of combing force.

**Claims**

1. Botanical mixture (BM) comprising the following 9 botanical components

    b1) bamboo extract,

b2) cactus extract,
b3) jojoba oil,
b4) safflower oil,
b5) olive oil,
b6) babassu oil,
b7) sesame seed oil,
b8) rice bran oil, and
b9) evening primrose oil.

2. The botanical mixture (BM) according to claim 1, wherein the botanical mixture (BM) comprises

1 to 10 % by weight of component b1),
1 to 10 % by weight of component b2),
10 to 30 % by weight of component b3),
10 to 30% by weight of component b4),
5 to 15 % by weight of component b5),
5 to 15 % by weight of component b6),
5 to 15 % by weight of component b7),
5 to 15 % by weight of component b8),
5 to 15 % by weight of component b9),

wherein the % by weight values are based on the total weight of the botanical mixture (BM), wherein the sum of components b1) to b9) is less than or equal to 100 % by weight of the botanical mixture (BM).

3. A hair cosmetic product comprising the botanical mixture (BM) according to claim 1 or 2.

4. The hair cosmetic product according to claim 3, wherein the hair cosmetic product comprises 0.05 wt.-% to 5.0 % by weight of the botanical mixture (BM) based on the total weight of the hair cosmetic product.

5. The hair cosmetic product according to claim 3 or 4, wherein the hair cosmetic product comprises at least one cosmetically acceptable aqueous or aqueous-organic medium.

6. The hair cosmetic product according to claim 5, wherein the hair cosmetic product comprises the at least one cosmetically acceptable aqueous or aqueous-organic medium in an amount in the range of 50 to 90 % by weight based on the total weight of the hair cosmetic product.

7. The hair cosmetic product according to any one of claims 3 to 6, wherein the hair cosmetic product comprises at least one fatty substance being different form the 9 botanical components comprised in the botanical mixture (BM).

8. The hair cosmetic product according to claim 7, wherein the hair cosmetic product comprises the at least one fatty substance in an amount in the range of 5 to 25 % by weight based on the total weight of the hair cosmetic product.

9. The hair cosmetic product according to any one of claims 3 to 8, wherein the hair cosmetic product comprises at least one surfactant.

10. The hair cosmetic product according to claim 9, wherein the hair cosmetic product comprises the at least one surfactant in an amount in the range of 5 to 25 % by weight based on the total weight of the hair cosmetic product.

11. The hair cosmetic product according to any one of claims 3 to 10, wherein the hair cosmetic product has a pH value in the range of 5.5 to 6.5.

12. The hair cosmetic product according to any one of claims 3 to 11, wherein the hair cosmetic product is selected from the group consisting of shampoos, rinse-off conditioners, hair masks, hair treatments, leave-in conditioners, hair serums, hair oils, hair creams, hair lotions, styling gels, styling creams, mousses, foams, heat protectant sprays, hair sprays, dry shampoos, hair pomades, hair waxes, hair butters, scalp treatments, scalp serums, anti-frizz sprays, anti-frizz creams, detangling sprays, hair perfumes, hair relaxers, hair straightening creams, hair dyes, hair coloring treatments, hair growth serums, curl enhancers, curl creams, split-end repair treatments, hair setting sprays, texturizing sprays and powders.

13. The hair cosmetic product according to any one of claims 3 to 12, wherein the hair cosmetic product is a shampoo, a conditioner, a serum or a hair mask comprising at least one cosmetically acceptable aqueous or aqueous-organic medium, at least one fatty substance, and at least one surfactant.

14. A method for treating hair comprising applying the botanical mixture (BM) according to claim 1 or 2 or the hair cosmetic product according to any one of claims 3 to 13 to a person's scalp hair.

15. Use of the botanical mixture (BM) according to claim 1 or 2 or the hair cosmetic product according to any one of claims 3 to 13 for nourishing and/or strengthening human hair.

## EUROPEAN SEARCH REPORT

**Application Number**

EP 25 21 8227

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; 2 October 2023 (2023-10-02), anonymous: "Wonder Balm", XP093274559, Database accession no. 11154364 * ingredient list, product description; the whole document * | 1-15 | INV. A61Q5/12 A61K8/92 A61K8/9789 A61K8/9794 |
| Y | DATABASE GNPD [Online] MINTEL; 5 December 2022 (2022-12-05), anonymous: "Instant Frizz Tamer Leave In Cream", XP093274560, Database accession no. 10374008 * ingredient list, product description; the whole document * | 1-15 | |
| Y | DATABASE GNPD [Online] MINTEL; 30 March 2010 (2010-03-30), anonymous: "Volume Boosting Shampoo", XP093274562, Database accession no. 1304422 * product description, ingredients list; the whole document * | 1-15 | TECHNICAL FIELDS SEARCHED (IPC) A61K A61Q |
| Y | KR 2020 0064464 A (DAMYANG JUKSOON FARMING ASSOCIATION CORP [KR]) 8 June 2020 (2020-06-08) * claims 1-7 * | 1-15 | |
| Y | CN 107 970 144 A (SHEN YANGYANG) 1 May 2018 (2018-05-01) * claims 1-2 * | 1-15 | |

The present search report has been drawn up for all claims

| Place of search | Date of completion of the search | Examiner |
|---|---|---|
| Munich | 16 April 2026 | Briand, Benoit |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 8227

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2026

| Patent document cited in search report | Publication date | Patent family member(s) | Publication date |
|---|---|---|---|
| KR 20200064464 A | 08-06-2020 | NONE | |
| CN 107970144 A | 01-05-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- International Cosmetics Ingredient Dictionary and Handbook. The Cosmetics, Toiletry, and Fragrance Association, vol. 2
- **AE MARTELL** ; **RM SMITH**. Critical Stability Constants. Plenum Press, 1974, vol. 1
- **AE MARTELL** ; **RD HANCOCK**. Metal Complexes in Aqueous Solution. Plenum Press, 1996
- **GARCIA** ; **DIAZ**. JSCC. *Combability Measurements on Hair*, 1976, vol. 27, 379-398